# EUROPEAN PATENT SPECIFICATION

(11) **EP 0 286 114 B1**
(45) Date of publication and mention of the grant of the patent: **30.03.1994**
(21) Application number: 88105623.8
(22) Date of filing: 08.04.1988
(51) Int. Cl.: C12N 15/09, C12N 15/10, C12N 15/11, C12N 15/12, C12P 21/02, C07K 13/00

(54) **Reg gene and protein encoded by the gene**
Reg-Gen und dadurch kodiertes Protein
Gène reg et protéine codée par ce gène

(30) Priority: 09.04.1987 JP 87807/87; 10.08.1987 JP 200514/87
(43) Date of publication of application: 12.10.1988
(73) Proprietor: SHIONOGI SEIYAKU KABUSHIKI KAISHA trading under the name of SHIONOGI & CO. LTD., Osaka 541 (JP)
(72) Inventor: Okamoto, Hiroshi, Sendai-shi Miyagi (JP)
(74) Representative: VOSSIUS & PARTNER

(56) References cited:
- EP-A- 0 258 000
- THE JOURNAL OF BIOLOGICAL CHEMISTRY, vol. 263, no. 5, 15th February 1988, pages 2111-2114, The American Society for Biochemistry and Molecular Biology, US; K. TERAZONO et al.: "A novel gene activated in regenerating islets"
- DIABETES, vol. 33, April 1984, pages 401-404; Y. YONEMURA et al.: "Amelioration of diabetes mellitus in partially depancreatized rats by poly(ADP-ribose) synthetase inhibitors. Evidence of islet B-cell regeneration"

## Description

This invention relates to the reg gene of mammals, especially of humans and rats, and to the encoded protein.

Insulin-producing pancreatic islet B cells have an extremely limited ability to regenerate, which creates a predisposition for the development of diabetes. In 1984, Okamoto and his colleagues successfully induced regeneration of pancreatic islet B cells for the first time by daily injection of poly(ADP-ribose) synthetase inhibitors such as nicotinamide to 90% depancreatized rats (Yonemura, Y. et al., Diabetes 33, 401, 1984). Additionally, it has recently been reported that the remission phase of human insulin-dependent diabetes can be prolonged by oral administration of nicotinamide in large doses (Ph. Vague et al., Lancet Vol. I, No. 8533, 619-620, 1987).

The differential hybridization method used in this invention has been developed by Takasawa et al. (Diabetes 35, 1178, 1986). That is, a gene expressed specifically in pancreatic B-cell tumors was successfully identified by differential screening of a rat B-cell tumor cDNA library using cDNA prepared from poly(A)⁺ RNA of B-cell tumors and cDNA from poly(A)⁺ RNA of normal pancreatic islets as probes.

Administration of insulin is the only therapy for diabetes currently recognized. Oral hypoglycemic agents such as sulfonylurea, which were once expected to be effective, have been found to exhibit adverse side effects, including disturbance of the ability of pancreatic B cells to produce insulin and the induction of coronary arteriosclerosis with long-term administration.

As described above, it was demonstrated that pancreatic islet B cells could be induced to regenerate in the remaining pancreas of 90% depancreatized rats receiving daily administration of poly(ADP-ribose) synthetase inhibitors such as nicotinamide. The molecular mechanism of this process has not yet been elucidated, however.

For the purpose of revealing the mechanism of the regeneration and proliferation of pancreatic islet B cells, it was searched for a gene that is specifically expressed in rat regenerating pancreatic islet B cells. The discovered gene was named "reg", i.e. regenerating gene. Furthermore, it was searched for a human reg gene by using the rat reg gene as a probe. A human reg DNA sequence was isolated from a human pancreatic cDNA library.

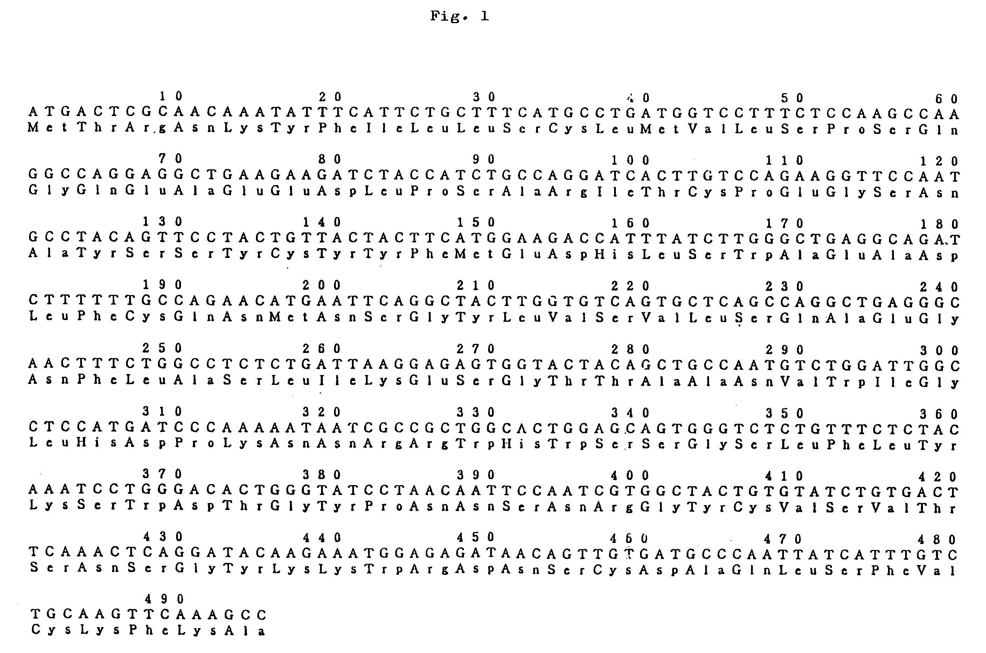

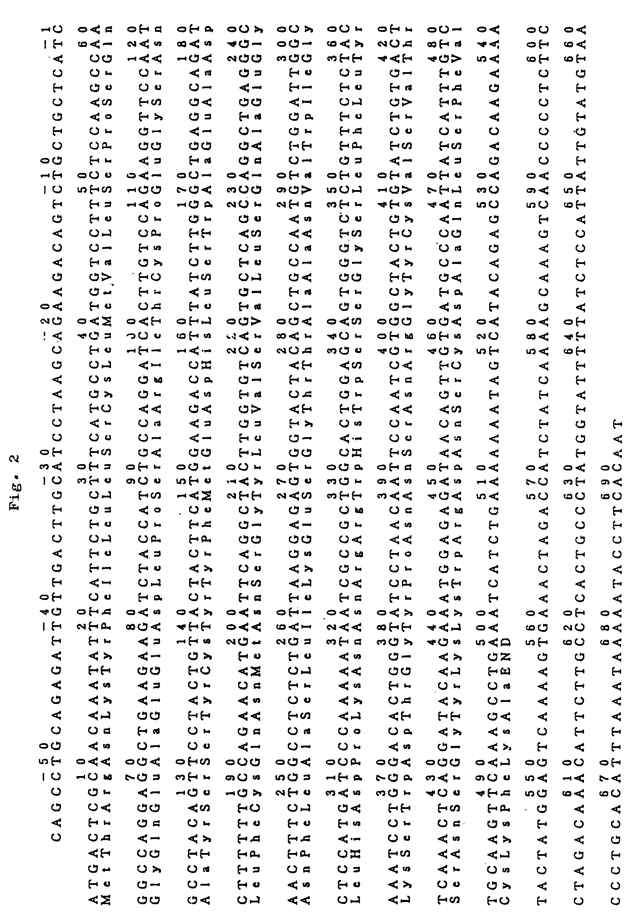

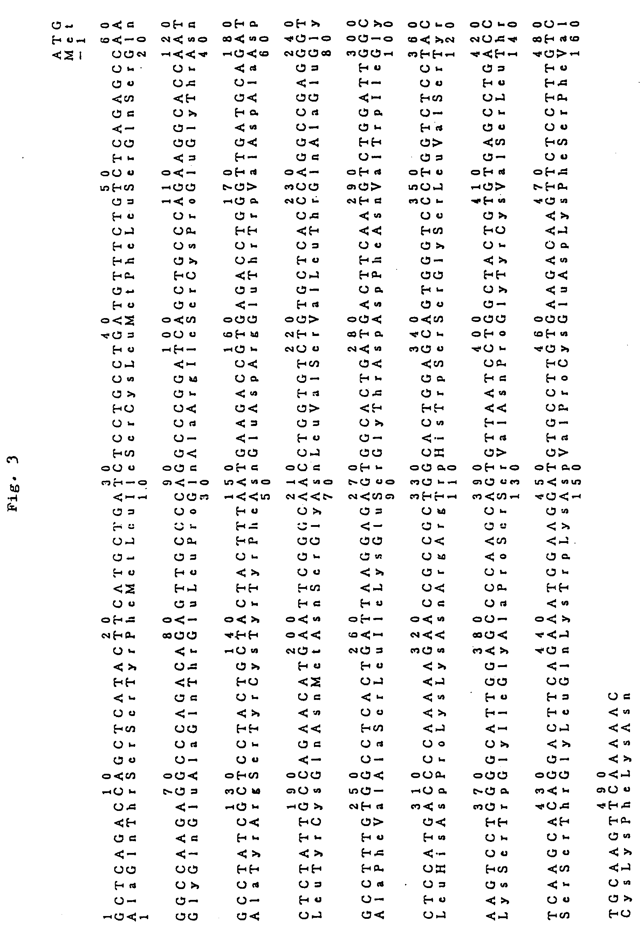

Fig. 1 shows the base sequence of the rat reg gene and the amino acid sequence deduced therefrom. Fig. 2 shows the whole base sequence of a rat reg cDNA and the amino acid sequence deduced therefrom. Fig. 3 shows the base sequence of a human reg cDNA and the amino acid sequence deduced therefrom.

The reg gene of this invention is considered to be closely related to the regeneration of insulin-producing pancreatic B cells. By mass-producing the proteins encoded by the gene and developing them into a clinically usable medicine, it may be possible to open a new dimension in the treatment of diabetes, surpassing conventional insulin administration therapy; that is, by the induction of the regeneration of insulin-producing pancreatic B cells of patients with diabetes.

In addition, though the mechanisms of the regeneration and proliferation of pancreatic B cells have not been fully understood, the isolation of the reg gene might well provide a clue toward the elucidation of the mechanisms at the cellular and molecular levels.

When the rat or human reg gene of the present invention are used as a probe, similar genes and their products can be detected in other mammalian species, which makes it possible to obtain the related genes and their products.

At first, rats are 90% depancreatized in order to obtain rat regenerating pancreatic B cells. This method was developed by V. G. Foglia (Rev. Soc. Argent. Biol. 20, 21-37, 1944). The rats fall into a diabetic state within a month after 90% pancreatectomy, but when the partially depancreatized rats receive daily injections of nicotinamide (about 0.5 g/kg weight daily) from a week before to 3 months after the operation, regeneration of islets is induced in the remaining pancreases, thereby leading to the prevention or amelioration of diabetes. The rat regenerating pancreatic islets are isolated by the known collagenase method (Okamoto, H. et al. FEBS Lett. 54, 103, 1975; Yamamoto, H., Okamoto, H., Lectures on Internal Secretion Experiment Vol. 3, 278, 1982, Kodansha Scientific, printed in Japan).

The isolated regenerating islets are homogenized and total RNA is recovered by gradient centrifugation according to the method of Chirgwin et al. (Biochemistry 1979: 18: 5294-99). From the obtained total RNA, poly(A)⁺ RNA is isolated by means of oligo(dT) cellulose chromatography according to the method of H. Aviv and P. Leder (Proc. Natl. Acad. Sci. 69, 1408-1412, 1972).

By using the isolated poly(A)⁺ RNA as a template, cDNA is synthesized with reverse transcriptase and the hybrid of cDNA and RNA is treated with RNaseH and DNA polymerase I to obtain double stranded cDNA. This process is carried out according to the method of U. Gubler et al. (Gene 25, 263-269, 1983). If a commercial kit for cDNA preparation (for example, cDNA synthesizing system produced by Amersham) is used, cDNA can easily be prepared.

The obtained double-stranded cDNA is inserted into an appropriate cloning vector and the obtained vector is further introduced into an appropriate host to be propagated, after which it is utilizable as a cDNA library of rat regenerating islets. Appropriate cloning vectors are phage vectors such as λ gt10 and plasmid vectors such as pNO1523, pKB111, pBR322, pUC, Okayama-Berg, Col El and Heidecher-Messing, and as appropriate hosts, E. coli K-12 HB101, C600, L87 and NM514 may be selected. Such vectors and hosts are commercially available. cDNA can be cloned without difficulty if commercial cDNA cloning systems (as manufactured by Amersham) are used.

In order to find the gene expressed specifically in rat regenerating pancreatic B cells, it is necessary at first to remove insulin cDNA clones from the cDNA library. This is because most of the mRNA present in B cells is synthesized from the insulin genes, which disturbs the screening of genes expressed specifically in the regenerating pancreatic B cells. A part of the cDNA library is hence fixed on nitrocellulose filters and hybridized to rat insulin cDNA (Metabolism and Disease Vol. 17, No. 7, 1185, 1980) and the non-insulin cDNA clones, which are not hybridized in this process, thus become available for the subsequent screening.

For the purpose of screening the genes expressed specifically in the regenerating islet B cells, a known differential screening method (Takasawa, S. et al., Diabetes 35, 1178, 1986) is adopted. That is, samples taken from the cDNA library are immobilized on two sets of nitrocellulose filters. Hybridization is performed by using the cDNA prepared from rat regenerating islet poly(A)⁺ RNA, put on one set of filters, and the cDNA prepared from normal islet poly(A)⁺ RNA, on the other set of filters as probes. The clone hybridizing only to the probe prepared from the regenerating islets is selected by comparing the autoradiograms of the two sets of filters, and a cDNA clone of the gene expressed specifically in the regenerating islet B cells is hence obtained.

Should this invention need to be reproduced, the screening can be performed with ease by using a probe prepared with reference to the base sequence shown in Fig. 1. Alternatively the DNA sequences of the present invention and their allelic derivatives can be obtained by in vitro DNA synthesis according to known methods.

In this invention, one kind of cDNA clone of the gene expressed specifically in the rat regenerating islet B cells could be obtained. As the gene is considered to be closely related to the regeneration of the islet B cells, it was named "reg gene". The base sequence of the cDNA was determined according to the method of Sanger et al. using the M13 system (Methods in Enzymology 101, 20-78, 1983) (Fig. 1). A variety of homology searches were performed on the base sequence and on the deduced amino acid sequence but none showed 50% or more homology, thus indicating that it was a completely novel DNA and a novel protein that were in question. Additionally, rat normal islets, liver, kidney, brain and insulinoma were analyzed for reg mRNA levels, which were found to be extremely low.

The rat reg protein of the present invention is not restricted to the protein having the amino acid sequence shown in Fig. 1: since the relevant activity is considered to be preserved, even with some slight substitution, elimination, insertion or addition of some amino acid residues, or even in a part of the protein, any proteins having the same activity are therefore included in the scope of this invention. Similarly, a rat reg gene is also not restricted to one having the base sequence shown in Fig. 1, and a variety of genes encoding various types of proteins, as stated above, are considered to be included within the scope of this invention.

Furthermore, the rat reg protein deduced has a lot of hydrophobic amino acid residues on the N-terminus, which is inferred to be a so-called signal sequence. Accordingly, the mature protein encoded by the rat reg seems to have a sequence starting with Gln at 22nd position and ending with Ala at 165th position in Fig. 1. Moreover, Met at the first position of the N-terminus may be removed from the protein in a cell and may not directly relate to the activity of the reg protein. Therefore, this invention includes any protein having the same acivity as the protein that starts from either Met (1st), Thr (2nd) or Gln (22nd) and ends with Ala (165th) as shown in Fig. 1. Similarly, the rat reg of this invention includes any gene that has the base sequence encoding the above 3 kinds of proteins, that is, the sequence that starts from either the first codon ATG, the 2nd codon ACT or the 22nd codon CAG and ends with the 165th codon GCC or a termination codon. When the rat reg protein is produced by genetic engineering techniques it may be preferable to use a base sequence having an initiation codon ATG (encoding Met) at the 5'-terminus and, therefore, this invention further includes genes in which ATG is connected to the above various genes on the 5ʹ-terminus and similarly includes proteins in which Met is added to the above various proteins on the N-terminus.

A human reg DNA sequence can be obtained from a human pancreatic cDNA library by utilizing a part of the base sequence of the rat reg as a probe.

A human pancreatic cDNA library is prepared in the following manner.

Human pancreas obtained surgically is homogenized and total RNA is recovered therefrom by density gradient centrifugation according to the method of Chirgwin et al (Biochemistry 1979; 18: 5294-99). From the obtained total RNA, poly(A)⁺ RNA is isolated by oligo(dT) cellulose chromatography according to H. Aviv and P. Leder (Proc. Natl. Acad. Sci. 69, 1408-1412, 1972).

From the isolated poly(A)⁺ RNA, cDNA is synthesized with reverse transcriptase, and the hybrid of cDNA and RNA is treated with RNaseH and DNA polymerase I to obtain double stranded cDNA. This process is carried out according to the method of U. Gubler et al. (Gene 25, 263-269, 1983). If a commercial kit for cDNA preparation (for example, the cDNA synthesizing system produced by Amersham) is used, cDNA can easily be prepared.

The obtained double-stranded cDNA is inserted into an appropriate cloning vector and the recombinant is further introduced into an appropriate host to be proliferated, after which it is utilizable as a human pancreatic cDNA library. Appropriate cloning vectors are phage vectors such as λ gt10 and plasmid vectors such as pNO1523, pKB111, pBR322, pUC, Okayama-Berg, Col El and Heidecher-Messing, and as appropriate hosts, E. coli K-12 HB101, C600, L87 and NM514 may be selected. Such vectors and hosts are commercially available. cDNA can be cloned without difficulty if commercial cDNA cloning systems (as manufactured by Amersham) are used.

Human reg DNA is detected from the obtained cDNA library by using a probe prepared from the rat reg gene described above. The probe is prepared in the following manner; the rat reg gene is digested with appropriate restriction enzymes or a suitable part of the rat reg gene is synthesized by an automated DNA synthesizer to obtain a DNA fragment, which is labeled with the Klenow fragment of DNA polymerase I or T4 polynucleotide kinase and with appropriate radiolabeled nucleotides. The probe is hybridized to the cDNA library. The clone with the longest DNA insert is selected from the positive clones and the DNA insert is isolate from the clone and sequenced. As a result, it is revealed that the gene shows 77% homology with the rat reg gene and therefore is determined to be the human reg gene. The amino acid sequences deduced from both genes have 68% homology. The base sequence of the human reg gene and the amino acid sequence deduced therefrom are shown in Fig. 3.

The amino acid sequence around the N-terminus deduced from the human reg gene is rich in hydrophobic amino acid residues and, accordingly, is inferred to be a signal sequence. Accordingly, the mature protein encoded by the human reg gene starts with either Gly (21st), Gln (22nd), Ala (24th), Gln (25th), Gln (30th) or Ala (31st) from among the amino acid residues in Fig. 3. The present invention also includes these mature proteins. When these proteins are produced by genetic engineering techniques, Met derived from an initiation codon may be added to the N-terminus of the proteins. The present invention includes such proteins that have Met at the N-terminus of the above proteins.

This invention also includes any gene encoding the amino acid sequence starting with Ala (1st), Gly (21st), Gln (22nd), Ala (24th), Gln (25th), Gln (30th) or Ala (31st) and ending with Asn (165th) from among the amino acid residues shown in Fig. 3, and any gene that has a base sequence starting with GCT (1st codon), GGC (21st codon), CAA (22nd codon), GCC (24th codon), CAG (25th codon), CAG (30th codon) or GCC (31st codon) and ending with AAC (165th codon) from among the nucleotide residues shown in Fig. 3. This invention further includes genes in which the initiation codon (ATG) and the termination codon (TAA, TAG or TGA) are connected to the genes described above on the 5'- and 3'-termini, respectively, and proteins in which the intiator Met is added to the proteins described above on the N-terminus. Furthermore, the present invention relates to the allelic derivatives of the identified reg DNA sequences. Still further the present invention relates to DNA sequences hybridising to the DNA sequences of Figures 1 to 3 under conventional conditions.

Finally, those connected to expression vectors and their protein products are also included in the invention.

The reg protein encoded by the human reg DNA sequence of this invention is not restricted to the protein that has the amino acid sequence shown in Fig. 3. Since the relevant activity is considered to be preserved, even with some slight substitution, elimination, insertion or addition of some amino acid residues, or even in a part of the protein, any proteins having the same activity are therefore included in the scope of this invention. Similarly, the human reg DNA sequence is also not restricted to one that has the base sequence shown in Fig. 3, and a variety of genes encoding various types of reg proteins as stated above are considered to be included within the scope of this invention.

The obtained cDNA can be expressed by conventional methods. For example, the cDNA can be expressed after it is inserted in an appropriate expression vector (pKK223-3, pBS, pDR540, pDR720, pPL-lambda etc.) under the control of an appropriate promoter (e.g. lac, tac, trp, p_{L}) and introduced in a host (E. coli K-12 AG-1, MM294, DH1, HB101, C600 etc.). When the reg-encoded protein is glycosylated, it is preferable to use eukaryotic cells such as yeast and monkey cells as a host. For example, when yeast is used, various known vectors such as pGPD-1, pMFα8, AAR6, ABade8, AH5, YEp52, pACF301 and pAM82 can be used as expression vectors. If the monkey cell COS is used, pKSV-10 and others are usable as expression vectors. Aside from the above host-vector systems, most of the known host-vector systems are suitable for the expression of reg proteins.

The expressed reg protein may be purified according to conventional purifying methods by appropriately combining centrifugation, chromatography, dialysis, salting-out and other methods.

Furthermore, this invention is not restricted to the human and the rat reg gene and the encoded proteins. Taking into consideration the fact that the base sequences of the human and rat reg gene and their deduced amino acid sequences show high homology, a reg-like gene must be found also in other species of mammals. Therefore, this invention includes reg genes and their expression products of other mammalian species. In other words, the present invention allows to isolate any gene which hybridizes to a probe prepared with reference to the base sequence of the human or rat reg gene of the present invention. Genes showing high homology with the present human or rat reg gene can easily be obtained from cDNA libraries of other mammalian pancreata by using the human or rat reg gene or portions of it as probes. It seems preferable to use as a probe a labeled DNA composed of the whole or a part (20-100 bases) of the human or rat reg gene of the present invention. Proteins encoded by such genes are, of course, included in the scope of this invention. Accordingly, the terms "reg" and "reg protein" as used according to the present invention do not only refer to the corresponding genes and proteins found in humans and rats but also to those found in other mammalian species.

### Example

### I . Isolation of rat regenerating pancreatic islets

### (1) 90% pancreatectomy

Male Wistar rats weighing 200 to 300 g were laparotomized under anesthesia with ether and the pancreas except for the parabiliary segment (corresponding to 90% of the whole pancreas) was resected, and the incised part was closed. The 90% pancreatectomy was established by V. G. Foglia (Rev. Soc. Argent. Biol. 20, 21-37, 1944) and it is widely used for the production of models of insulin-dependent diabetes. The rats fall into a diabetic state within a month after being subjected to 90% pancreatectomy.

### (2) Administration of nicotinamide

Nicotinamide (50 mg/ml physiological saline) was intraperitoneally administered once a day at a dose of 0.5 g/kg body weight from 7 days before to 3 months after the 90% pancreatectomy. The administration of nicotinamide prevented and ameliorated the diabetic status of the 90% depancreatized rats.

### (3) Isolation of regenerating islets

By the above steps (1) and (2), the regeneration of islets was induced in the remaining pancreas and both the number of islets per unit volume of pancreas and the size per islet increased, the increase in the number of islet cells being responsible for that in their size. Most of the increasing cells were insulin-producing B cells and the numbers of glucagon-producing A cells and somatostatin-producing D cells were not changed (Yonemura, Y. et al. Diabetes 33, 401, 1984). Three months after the operation the 90% depancreatized and nicotinamide-treated rats were laparotomized under anesthesia with Nembutal and the remaining pancreas was removed after being swollen with Hanks' solution. The pancreata were comminuted in 5 ml of Hank's solution and per two used pancreata, 150 mg of bovine serum albumin (Sigma, Type V) and 40 mg of collagenase (Wako Pure Chemical Industries Ltd., Type IV) were added, and the mixture was shaken for 3 to 5 minutes at 37°C for digestion. After the digestion, the solution was suspended in 50 ml of Hanks' solution and allowed to stand for 5 minutes for the islets to be precipitated. Then the upper phase of the Hanks' solution was removed and a further 25 ml of fresh Hanks' solution was added, the mixture was suspended again. After standing, removal and resuspension had been performed 8 times, the regenerating islets were collected under a stereoscopic microscope. Then, 1355 regenerating islets were collected from 32 rats which bad been 90%-depancreatized and nicotinamide- treated. The above method basically conforms to the method reported by Yamamoto and Okamoto (Lecture of Internal Secretion Experiment Vol. 3, 278, 1982, Kodansha Scientific, printed in Japan).

### II. Preparation of a rat regenerating pancreatic islet cDNA library

### (1) Isolation of total RNA

Isolated regenerating islets were disrupted at 4°C by sonication in 4M guanidinium isothiocyanate, 10mM sodium citrate (pH 7.0), 0.5% sodium lauryl sarcosine, 0.1 M 2-mercaptoethanol and 1% Antifoam A (Sigma). The mixture was layered onto cesium trifluoroacetate with a density of 1.64 (Pharmacia) and centrifuged for 14 to 16 hours at 25°C at 180 000 xg. After the centrifugation, the RNA precipitate was recovered. From 1355 regenerating islets, 885 µg of total RNA was obtained (Chirgwin, J. M. et al., Biochemistry 1979, 18: 5294-99).

### (2) Isolation of poly(A)⁺ RNA

From the total RNA obtained in the above step (1), 17.2 µg of poly(A)⁺ RNA was isolated by oligo(dT) cellulose column chromatography (H. Aviv. & P. Leder, Proc. Natl. Acad. Sci. 69, 1408-1412, 1972).

### (3) Preparation of cDNA library

At first, by using 2 µg of poly(A)⁺ RNA from regenerating islets as a template and oligo(dT) as a primer and by incubating them with 40 units of reverse transcriptase for an hour at 42°C, 182 ng of cDNA (first strand) was synthesized. The obtained cDNA-RNA hybrid was treated with 0.75 units of RNaseH, 46 units of DNA polymerase I and 4 units of T4 DNA polymerase to obtain 284 ng of double-stranded cDNA. The synthesis of cDNA stated above was carried out basically according to the method of U. Gubler et al. (Gene 25, 263-269, 1983).

cDNA (double-stranded) was treated with 15 units of EcorRI methylase (New England Biolabs) and ligated to an EcoRI linker (450 ng) with 175 units of T4 DNA ligase (Takara Shuzo) for 45 hours at 13°C.

The above product was digested with 76 units of EcoRI (Takara Shuzo) for 2 hours at 37°C and applied onto Sephacryl S-1000 (Pharmacia) to be separated into cDNA and digested linker. By the above steps, 214 ng of EcoRI methylase-treated EcoRI fragment (double-stranded cDNA) was recovered.

From the above cDNA, 20 ng was ligated with 1.23 µg of λ gt10 arms (dephosphorylated at 5ʹ terminal by alkaline phosphatase after being digested by EcoRI, Stratagene) with the use of T4 DNA ligase (Takara Shuzo, 116 units) for 15 hours at 13°C. The ligated λ gt10-regenerating islet cDNA was packaged with the use of Gigapack (Stratagene) and introduced into E. coli K12-derived Y1089 (DNA cloning, vol. 1, 49-78, 1985, IRL Press) and thus a total of 2.8 x 10⁵ transformants were obtained.

### III. Differential screening of regenerating islet cDNA library:

### Isolation of genes specifically expressed in regenerating islets

From each of 5000 independent plaques arbitrarily selected from regenerating islet cDNA libraries prepared in the above step II, phages were inoculated with toothpicks into NZY-0.2% maltose medium (1% NZ amine, 0.5% Bacto-yeast extract, 0.5% sodium chloride and 0.2% maltose) containing Y1089 corresponding to 0.5 OD₆₀₀ in microtiter plates (96 wells) and incubated overnight at 37°C for proliferation. Two µl of each phage solution obtained was spotted onto nitrocellulose filters (Schleicher and Schüll, BA85) and hybridized to rat insulin II cDNA labeled with ³²P by nick translation. As a result, 16% of the clones were identified as insulin cDNA clones and the remaining 84% non-insulin cDNA clones were available for the subsequent screening.

Poly(A)⁺ RNA (150 ng) from rat regenerating islets or from untreated rat normal islets, was incubated for 2 hours at 37°C with 15 units of reverse transcriptase (Seikagaku Kogyo) in the presence of 41 ng of oligo(dT)₁₂₋₁₈, 20 mM dATP, 20 mM dTTP, 20 mM dGTP, 2 µM dCTP and 60 µCi[α-³²P]dCTP (Amersham). Thereafter the RNA chain was degraded with 0.3N sodium hydroxide (at 100°C for 2 min.), and the produced ³²P- labeled single-stranded cDNA was recovered by means of ethanol precipitation. By this method, probes having a specific activity of 1 to 2 x 10⁸ cpm/µg poly(A)⁺ RNA were obtained.

Two sets of nitrocellulose filters were spotted with 2 µl samples of each phage solution of non-insulin cDNA clones, treated at room temperature with 0.5 M sodium hydroxide/1.5 M sodium chloride for 5 minutes and then with 0.5 M tris-hydrochloric acid (pH 8.0)/1.5 M sodium chloride for another 5 minutes, then dipped in 2 x SSC (1 x SSC: 0.15 M sodium chloride and 0.015 M sodium citrate) and baked for 2 hours at 80°C.

The baked nitrocellulose filters were allowed to react in 3 x SSC for 30 minutes at 65°C and then in 3 x SSC and 1 x FBP (Denhardt's solution; 0.02% Ficoll 400, 0.02% bovine serum albumin and 0.02% polyvinyl pyrrolidone) for an hour at 65°C and pre-hybridized at 65°C for an hour in 1 M sodium chloride, 50 mM tris-hydrochloric acid (pH 7.4), 10 mM EDTA, 0.1% SDS, 1 x FBP, 10 µg/ml salmon testis DNA and 250 µg/ml poly(U) (Pharmacia). Hybridization was carried out with the use of cDNA (1.5 x 10⁷ cpm) prepared from rat regenerating islet poly(A)⁺ RNA as a probe for one set of filters (stated before) and cDNA (1.5 x 10⁷ cpm) prepared from normal islet poly(A)⁺ RNA isolated from untreated rats as a probe (stated before) for the other set of filters in 1 M sodium chloride, 50 mM tris-hydrochloric acid (pH 7.4), 10 mM EDTA (pH 8.0), 0.1% SDS and 250 µg/ml poly(U) for 16 hours at 65°C.

After the hybridization, the filters were washed twice with 2 x SSC at room temperature for 1 to 2 minutes and further washed 4 times with 0.1 x SSC/0.1% SDS at 65°C for 40 minutes and dried at 80°C for an hour and the autoradiography was carried out at -80°C overnight. Consequently, one kind of clone that consistently hybridized specifically with regenerating islet cDNA could be obtained.

### IV. Sequencing of the gene specifically expressed in the rat regenerating islet B cells

The DNA of the phage cDNA clone which was obtained in the above step III and which comprises the gene specifically expressed in the regenerating islets, was prepared (see T. Maniatis et al., Molecular Cloning, A Laboratory Manual, 1982, 80 and seq.) and digested with EcoRI. The cDNA insert was separated from the phage vector arms by 1% agarose gel electrophoresis. The base sequence of the isolated cDNA was determined by Sanger's method using M13 system (mp18 and mp19, Pharmacia)

### (Methods in Enzymology 101, 20-78, 1983).

As a result, it was found out that the transcript expressed specifically in regenerating islets comprises a total length of 749 nucleotides, not including the poly(A) parts, and encodes a protein composed of 165 amino acid residues starting with methionine and ending with alanine. The whole base sequence and deduced amino acid sequence are shown in Fig. 2.

Computer analysis of the nucleic acid and protein data bases (method of Wibur, W. J. & Lipman, D. J., Proc. Natl. Acad. Sci. USA 1983, 80, 726-730) of European Molecular Biology Laboratory (Heidelberg), GenBank (Cambridge, Massachusetts) and National Biomedical Research Foundation (Washington, DC) revealed that the base sequence and deduced amino acid sequence of the newly cloned cDNA of this invention were different from any known genes or gene products, and no gene or gene product showing 50% or more homology could be found in the data bases.

Accordingly, the cDNA cloned from the regenerating islet cDNA library is considered to correspond to a completely new gene which has never been reported before and the gene was named "reg" (regenerating gene).

### V. Investigation of reg mRNA

RNA was isolated from rat regenerating islets and normal islets by the method described in II-(1), and 4 µg of RNA from each source was denatured by 1.1 M formalin, subjected to 1.5% agarose gel electrophoresis containing 1.1 M formalin and transferred onto a nitrocellulose filter. The filter was baked for at 80°C 2 hours, prehybridized at 42°C for 4 hours in 50% formamide, 5 x FBP, 5 x SSPE [1 x SSPE: 0.18 M sodium chloride, 10 mM sodium phosphate buffer (pH 7.7) and 1 mM EDTA), 250 µg/ml salmon testis DNA and 0.1% SDS, successively hybridized at 42°C for 20 hours in 50% formamide, 1 x FBP, 5 x SSPE, 0.1% SDS and 100 µg/ml salmon testis DNA solution containing reg cDNA (20 ng/ml) labeled with ³²P by nick translation, then washed twice at room temperature for 30 minutes in 2 x SSC/0.1% SDS, and twice at 37°C for an hour in 0.1 x SSC/0.1% SDS, and finally subjected to autoradiography (-80°C).

The results showed that reg mRNA had a chain length of about 900 nucleotides and was present in abundance in the regenerating islets but extremely scarce in the normal islets.

When the mRNA in rat liver, kidney, brain and insulinoma (Streptozotocin-induced) was similarly analyzed the levels of reg mRNA in these tissues were found to be extremely low.

### Example 2

### I. Isolation of human reg

### 1. Preparation of a human pancreatic cDNA library

a) It is impossible to prepare human regenerating islets of Langerhans and therefore a cDNA library was constructed from human pancreatic tissue that had been obtained by operation. In rat pancreatic tissue, reg mRNA level was low but still detectable, and therefore it was assumed that human pancreatic tissue would exhibit similar characteristics.
b) Isolation of total RNA
   Human pancreas obtained surgically was disrupted at 4°C with Polytron in 4M guanidinium isothiocyanate, 10mM sodium citrate (pH 7.0), 0.5% sodium lauryl sarcosine, 0.1 M 2-mercaptoethanol and 1% Antifoam A (Sigma). The mixture was layered onto cesium trifluoroacetate with a density of 1.64 (Pharmacia) and centrifuged for 14 to 16 hours at 25°C at 180 000 xg. After the centrifugation, RNA precipitate was recovered. From 500 mg of pancreatic tissue, 2.526 mg of RNA was obtained (Chirgwin, J. M. et al., Biochemistry 1979, 18: 5294-99).
c) Isolation of poly(A)⁺ RNA
   From the total RNA obtained in the above step (b), 17.56 µg of poly(A)⁺ RNA was isolated by oligo(dT) cellulose column chromatography (H. Aviv. & P. Leder, Proc. Natl. Acad. Sci. 69, 1408-1412, 1972).
d) Preparation of cDNA library
   At first, by using 1 µg of human pancreatic poly(A)⁺ RNA as a template and oligo(dT) as a primer and by incubating them with 40 units of reverse transcriptase for an hour at 42°C, about 100 ng of cDNA (first strand) was synthesized. The obtained cDNA-RNA hybrid was treated with 0.75 units of RNaseH, 46 units of DNA polymerase I and 4 units of T4 DNA polymerase to obtain about 150 ng of double-stranded cDNA. The synthesis of cDNA described above was carried out basically according to the method of U. Gubler et al. (Gene 25, 263-269, 1983).
   cDNA (double-stranded) was teated with 15 units of EcoRI methylase (New England Biolabs) and ligated to an EcoRI linker (450 ng) with 175 units of T4 DNA ligase (Takara Shuzo) for 45 hours at 13°C.
   The above product was digested with 76 units of EcoRI (Takara Shuzo) for 2 hours at 37°C and applied onto Sephacryl S-1000 (Pharmacia) to be separated into cDNA and digested linker. By the above steps, 17.6 ng of EcoRI methylase-treated EcoRI fragment (double-stranded cDNA) was recovered.
   The above cDNA was ligated to 1.0 µg of λ gt10 arms (dephosphorylated at 5ʹ terminal by alkaline phosphatase after having been digested with EcoRI, Stratagene) with T4 DNA ligase (Takara Shuzo, 116 units) for 15 hours at 13°C. The ligated λ gt10-human pancreatic cDNA was packaged with Gigapack (Stratagene), introduced into E. coli K12-derived Y1089 (DNA cloning, vol. 1, 49-78, 1985, IRL Press) and thus a total of 6 x 10⁵ transformants were obtained.

### 2. Cloning of human reg cDNA from human pancreatic cDNA library

a) From each plaque of the human pancreatic cDNA library prepared, phages were inoculated into maltose medium (0.2% maltose, 1% NZ amine, 0.5% Bacto-yeast extract and 0.5% sodium chloride) containing Y1089 corresponding to 0.5 OD₆₀₀ and incubated overnight at 37°C for proliferation. As a result, 10⁶ plaques were formed on an agar medium (150 mm in diameter) and transferred onto nitrocellulose filters.
b) Oligodeoxyribonucleotide (60 bases) corresponding to the base sequence from 76th to 135th of rat reg (Fig. 1) was synthesized by a DNA synthesizer (Applied Biosystems Model 380 B) and its 5' terminal was labelled with ³²P by using [γ-³²P] ATP and T4 polynucleotide kinase.
c) Nitrocellulose filters obtained in the above step a) were treated at room temperature with 0.5 N sodium hydroxide/1.5 M sodium chloride for 5 minutes and then with 0.5 M tris-hydrochloric acid (pH 8.0)/1.5 M sodium chloride for another 5 minutes, then dipped in 2 x SSC (1 x SSC: 0.15 M sodium chloride and 0.015 M sodium citrate) and baked for 2 hours at 80°C.
   The baked nitrocellulose filters were pre-hybridized at 50°C for 4 hours in 6 × SSC, 5 × FBP (1 × FBP; 0.02% Ficoll 400, 0.02% bovine serum albumin and 0.02% polyvinyl pyrrolidone), 0.1% SDS and 200 µg/ml E. coli tRNA, hybridized with the ³²P-labeled oligodeoxyribonucleotide at 50°C for 12-16 hours in 6 × SSC, 1 × FBP, 0.1% SDS and 100 µg/ml E. coli tRNA containing 2 ng/ml ³²P-labeled oligodeoxyribonucleotide and washed 4 times, 30 minutes each time, at 50°C with 6 × SSC and 0.1% SDS.
d) As a result, 58 clones among 10⁶ human pancreatic cDNA clones were hybridized with a rat reg probe of 60 bases.
e) From the positive clones, the clone with the longest cDNA insert (about 900 nucleotides) was selected. The DNA insert was isolated from the clone, digested with EcoRI and then sub-cloned in pBS vector (Stratagene) at EcoRI site.

### II. Sequencing of the human reg cDNA

a) The base sequence of the cDNA sub-cloned in pBS vector was determined according to Sanger' s method (Methods in Enzymology 101, 20-78, 1983).
b) As a result, it was found that the human reg cDNA comprises a total length of 749 nucleotides, not including the poly(A) part, and encodes a protein composed of 166 amino acid residues starting with methionine and ending with asparagine.
c) The protein encoded by the human reg is longer by one amino acid residue than the rat reg-encoded protein composed of 165 amino acid residues. The base sequence of the coding region of the human reg and the amino acid sequence deduced therefrom show 75% and 68% homologies with those of the rat reg, respectively.
d) The sequence around the N-terminus of the human reg protein is rich in hydrophobic amino acid residues. This is the main characteristic of the signal peptides of secretory proteins. Consequently, in the human reg protein the sequence starting with Met (-1) and ending with either Gln (20), Gly (21), Glu (23), Ala (24), Pro (29) or Ser (30) was deduced to be a signal peptide.

## Claims

1. A reg (regeneration) gene hybridizing to a probe corresponding to at least a part of the whole base sequence encoding the amino acid sequence shown in Fig. 1 (rat reg protein) or Fig. 3 (human reg protein).

2. The gene of claim 1, wherein said base sequence comprises the base sequence shown in Fig. 1 (rat reg gene) or Fig. 3 (human reg gene).

3. The gene of claim 1 or 2, wherein said gene is expressed in a mammalian pancreas.

4. The gene according to any one of claims 1 to 3, wherein said mammal is either human or rat.

5. The gene according to any one of claims 1 to 4 which encodes a protein that contains the amino acid sequence shown in Fig. 1.

6. The gene according to any one of claims 1 to 5, wherein said amino acid sequence starts with either Met (1st), Thr (2nd), Gln (22nd) or Met-Gln (22nd) and ends with Ala (165th) as in Fig. 1.

7. The gene according to any one of claims 1 to 6, which contains the base sequence shown in Fig. 1.

8. The gene according to any one of claims 1 to 7, wherein said base sequence starts with either ATG (1st codon), ACT (2nd codon), CAG (22nd codon) or ATG-CAG (22nd codon) and ends with either GCC (165th codon) or the termination codon shown in Fig. 1.

9. The gene according to any one of claims 1 to 4 and 8 which encodes a protein that contains the amino acid sequence shown in Fig. 3.

10. The gene according to any one of claims 1 to 4 and 8 to 9, wherein said amino acid sequence starts with Ala (1st), Gly (21st), Gln (22nd), Ala (24th), Gln (25th), Gln (30th) or Ala (31st) and ends with Asn (165th) as shown in the amino acid sequence of Fig. 3.

11. The gene according to any one of claims 1 to 4 and 8 to 10, wherein said base sequence comprises GCT (1st codon), GGC (21st codon), CAA (22nd codon), GCC (24th codon), CAG (25th codon), CAG (30th codon) or GCC (31st codon) to AAC (165th codon) as shown in the base sequence of Fig. 3.

12. The gene according to any one of claims 1 to 11, which additionally contains an initiation codon on the 5'-terminus and/or a termination codon at the 3'-terminus.

13. A recombinant DNA molecule containing a gene according to any one of claims 1 to 12.

14. A host organism which is transformed with a recombinant DNA molecule according to claim 13.

15. A protein encoded by the gene of any one of claims 1 to 12.

16. A method for preparing a gene according to any one of claims 1 to 12 hybridizing to a probe corresponding to a part or to the whole of a base sequence encoding the amino acid sequence shown in Fig. 1 or Fig. 3, which comprises preparing a mammalian pancreatic cDNA library and selecting the gene with the use of said probe.

17. A method for preparing a protein of claim 15 which comprises introducing a vector carrying a gene of any one of claims 1 to 12 under the control of an appropriate promoter into a suitable host, cultivating said host in a medium and recovering said protein from the culture.

## Patentansprüche

1. Reg (Regenerations)-Gen, mit einer Sonde hybridisierend, die mindestens einem Teil der gesamten Basensequenz entspricht, die die in Fig. 1 (Ratten-Reg-Protein) oder Fig. 3 (menschliches Reg-Protein) gezeigte Aminosäuresequenz codiert.

2. Gen nach Anspruch 1, wobei die Basensequenz die in Fig. 1 (Ratten-Reg-Gen) oder Fig. 3 (menschliches Reg-Gen) gezeigte Basensequenz umfaßt.

3. Gen nach Anspruch 1 oder 2, wobei das Gen in einem Säugerpankreas exprimiert wird.

4. Gen nach einem der Ansprüche 1 bis 3, wobei der Säuger entweder Mensch oder Ratte ist.

5. Gen nach einem der Ansprüche 1 bis 4, ein Protein codierend, das die in Fig. 1 gezeigte Aminosäuresequenz enthält.

6. Gen nach einem der Ansprüche 1 bis 5, wobei die Aminosäuresequenz wie in Fig. 1 entweder mit Met (1.), Thr (2.), Gln (22.) oder Met-Gln (22.) beginnt und mit Ala (165.) aufhört.

7. Gen nach einem der Ansprüche 1 bis 6, das die in Fig. 1 gezeigte Basensequenz enthält.

8. Gen nach einem der Ansprüche 1 bis 7, wobei die Basensequenz entweder mit ATG (1. Codon), ACT (2. Codon), CAG (22. Codon) oder ATG-CAG (22. Codon) beginnt und entweder mit GCC (165. Codon) oder dem in Fig. 1 gezeigten Terminationscodon aufhört.

9. Gen nach einem der Ansprüche 1 bis 4 und 8, das ein die in Fig. 3 gezeigte Aminosäuresequenz enthaltendes Protein codiert.

10. Gen nach einem der Ansprüche 1 bis 4 und 8 bis 9, wobei die Aminosäuresequenz, wie in der Aminosäuresequenz von Figur 3 gezeigt, mit Ala (1.), Gly (21.) Gln (22.), Ala (24.), Gln (25.), Gln (30.) oder Ala (31.) beginnt und mit Asn (165.) aufhört.

11. Gen nach einem der Ansprüche 1 bis 4 und 8 bis 10, wobei die Basensequenz wie in der Basensequenz von Fig. 3 gezeigt, GCT (1. Codon), GGC (21. Codon), CAA (22. Codon), GCC (24. Codon), CAG (25. Codon), CAG (30. Codon) oder GCC (31. Codon) bis AAC (165. Codon) umfaßt.

12. Gen nach einem der Ansprüche 1 bis 11, das außerdem ein Initiationscodon am 5'-Ende und/oder ein Terminationscodon am 3'-Ende enthält.

13. Rekombinantes DNA-Molekül, das ein Gen nach einem der Ansprüche 1 bis 12 enthält.

14. Wirtsorganismus, der mit einem rekombinanten DNA-Molekül nach Anspruch 13 transformiert ist.

15. Protein, das von dem Gen nach einem der Ansprüche 1 bis 12 codiert wird.

16. Verfahren zur Herstellung eines Gens nach einem der Ansprüche 1 bis 12, das mit einer Sonde hybridisiert, die einem Teil oder der gesamten Basensequenz entspricht, die die in Fig. 1 oder Fig. 3 gezeigte Aminosäuresequenz codiert, umfassend die Herstellung einer SäugerpankreascDNA-Genbank und die Selektion des Gens unter Verwendung dieser Sonde.

17. Verfahren zur Herstellung eines Proteins nach Anspruch 15, umfassend das Einschleusen eines Vektors, der ein Gen nach einem der Ansprüche 1 bis 12 unter der Kontrolle eines geeigneten Promotors trägt, in einen geeigneten Wirt, die Züchtung des Wirts in einem Medium und die Gewinnung des Proteins aus der Kultur.

## Revendications

1. Gène reg (de régénération) s'hybridant à une sonde correspondant à au moins une partie de la séquence de base complète codant la séquence d'acide aminé montrée sur la figure 1 (protéine reg de rat) ou la figure 3 (protéine reg humaine).

2. Le gène de la revendication 1, dans lequel la séquence de base comprend la séquence de base montrée sur la figure 1 (gène reg de rat) ou sur la figure 3 (gène reg humain).

3. Gène selon la revendication 1 ou 2, dans lequel le gène est exprimé dans un pancréas de mammifère.

4. Gène selon l'une quelconque des revendications 1 à 3, dans lequel le mammifère est soit l'homme soit le rat.

5. Gène selon l'une quelconque des revendications 1 à 4 qui code une protéine qui contient la séquence d'acide aminé montrée sur la figure 1.

6. Gène selon l'une quelconque des revendications 1 à 5, dans lequel la séquence d'acide aminé commence soit par Met (1^{er}) Thr (2^{ème}), Gln (22^{ème}) soit par Met-Gln (22^{ème}) et se termine par Ala (165^{ème}) comme sur la figure 1.

7. Gène selon l'une quelconque des revendications 1 à 6 qui contient la séquence de base montrée sur la figure 1.

8. Gène selon l'une quelconque des revendications 1 à 7, dans lequel la séquence de base commence soit par ATG (1^{er} codon), ACT (2^{ème} codon), CAG (22^{ème} codon) soit par ATG-CAG (22^{ème} codon) et se termine soit par GCC (165^{ème} codon) soit par le codon de terminaison montré sur la figure 1.

9. Gène selon l'une quelconque des revendications 1 à 4 et 8 qui code une protéine qui contient la séquence d'acide aminé montrée sur la figure 3.

10. Gène selon l'une quelconque des revendications 1 à 4 et 8 à 9, dans lequel la séquence d'acide aminé commence par Ala (1^{er}), Gly (21^{ème}), Gln (22^{ème}), Ala (24^{ème}), Gln (25^{ème}), Gln (30^{ème}) ou Ala (31^{ème}) et se termine par Asn (165^{ème}) comme cela est montré dans la séquence d'acide aminé de la figure 3.

11. Gène selon l'une quelconque des revendications 1 à 4 et 8 à 10, dans lequel la séquence de base comprend GCT (1^{er} codon), GGC (21^{ème} codon), CAA (22^{ème} codon), GCC (24^{ème} codon), CAG (25^{ème} codon), CAG (30^{ème} codon) ou GCC (31^{ème} codon) jusqu'à AAC (165^{ème} codon) comme cela est montré dans la séquence de base de la figure 3.

12. Gène selon l'une quelconque des revendications 1 à 11 qui contient de plus un codon d'initiation sur l'extrémité 5'-terminale et/ou un codon de terminaison en 3'-terminale.

13. Molécule d'ADN recombiné contenant un gène selon l'une quelconque des revendications 1 à 12.

14. Organisme-hôte qui est transformé avec une molécule d'ADN recombiné selon la revendication 13.

15. Protéine codée par le gène de l'une quelconque des revendications 1 à 12.

16. Procédé pour la préparation d'un gène selon l'une quelconque des revendications 1 à 12 s'hybridant à une sonde correspondant à une partie ou à la totalité d'une séquence de base codant la séquence d'acide aminé montrée sur la figure 1 ou la figure 3 qui comprend les opérations consistant à préparer une banque d'ADNc pancréatique de mammifère et à sélectionner le gène avec l'utilisation de la sonde.

17. Procédé pour la préparation d'une protéine selon la revendication 15 qui comprend les opérations consistant à introduire un vecteur portant un gêne de l'une quelconque des revendications 1 à 12 sous le contrôle d'un promoteur approprié dans un hôte adéquat, à cultiver l'hôte dans un milieu et à récupérer la protéine de la culture.
